# EUROPEAN PATENT SPECIFICATION

(11) **EP 1 340 490 B1**
(45) Date of publication and mention of the grant of the patent: **08.10.2008**
(21) Application number: 03425040.7
(22) Date of filing: 28.01.2003
(51) Int. Cl.: A61K 8/43, A61Q 11/00

(54) **Chlorhexidine-based collutory for oral hygiene**
Mund- und Gurgellösung auf Chlorhexidinbasis zur oralen Hygiene
Collutoire à base de chlorhexidine pour l'hygiène orale

(30) Priority: 01.03.2002 IT MI20020421
(43) Date of publication of application: 03.09.2003
(73) Proprietor: Castellaccio, Restituta, 21047 Saronno (VA) (IT)
(72) Inventor: Castellaccio, Restituta, 21047 Saronno VA (IT); Giovannardi, Stefano, 21047 Saronno VA (IT)
(74) Representative: Pizzoli, Pasquale Vincenzo

(56) References cited:
- EP-A- 0 804 920
- FR-M- 6 300
- US-A- 4 213 961
- DATABASE WPI Section Ch, Week 199421 Derwent Publications Ltd., London, GB; Class B05, AN 1994-175141 XP002244604 & SU 1 803 110 A (PHARMACY RES INST), 23 March 1993 (1993-03-23)
- PATENT ABSTRACTS OF JAPAN vol. 015, no. 488 (C-0893), 11 December 1991 (1991-12-11) & JP 03 215411 A (KANEBO LTD), 20 September 1991 (1991-09-20)
- DATABASE WPI Section Ch, Week 199930 Derwent Publications Ltd., London, GB; Class B05, AN 1999-352768 XP002244605 & JP 11 130648 A (LION CORP), 18 May 1999 (1999-05-18)

## Description

The present invention relates to a chlorhexidine-based collutory for oral hygiene and in particular to a collutory that prevents the plaque formation on teeth by avoiding the known drawback of the dark pigmentation thereof.

Solutions for oral hygiene based on chlorhexidine are already known since this compound is a strong antibacterial agent thanks to its ability to penetrate the outer membrane of bacteria and to coagulate their internal proteins. The chlorhexidine performs even an intense antiplaque activity. In fact it competes with calcium ions thus preventing the formation of the cuticle, the agglutination of bacteria, their adhesion to cuticle, the bond between glycoproteins and thus the formation of the bacterial plaque. Solutions containing chlorhexidine in different concentrations are used also to prevent postoperative complications. The use of solutions based on chlorhexidine in oral hygiene has however the known drawback of forming a brownish pigmentation on teeth.

To prevent this dark pigmentation of teeth it has already been proposed to add specific amounts of an organic acid such as citric or ascorbic acid to the chlorhexidine solution. For example, solutions of this kind are described in USP 4,213,961 as well as in EP 0804920A3 and in Japanese published application 3215411. French patent 6300M describes a collutory containing ascorbic acid as reducing agent for ryfamicine and JP 11130048 discloses the use of sodium metabisulphite in a medicament for treating periodontitis. Even though these solutions produce excellent results from every point of view, however they do not allow to eliminate the brownish pigmentation that develops on teeth by using the chlorhexidine solution in oral hygiene.

An object of the present invention is therefore to provide a new solution for oral hygiene based on chlorhexidine and ascorbic acid, which is free from the above-mentioned drawbacks. This object is achieved by adding sodium metabisulphite to the collutory, according to claim 1. Further features of the collutory are specified in the subsequent claims.

Even if the etiology of the external teeth pigmentation caused by chemical agents used to prevent the plaque formation is not well understood, recent experimental researches refer to two possible mechanisms: non enzymatic browning reactions and formation of pigmenting metal (Fe) sulphides. Such mechanisms, in turn, may be modified even by reaction products between constituents of foods and beverages and antiplaque chemical compounds.

The non enzymatic browning reactions, known as Maillard reactions, use, as a substrate, carbohydrates, peptides and protides which undergo a series of condensation and polymerization reactions leading to the formation of brown pigmenting substances, known as melanoidins. It has been found that such reactions are catalyzed by chlorhexidine, while they are inhibited by sulphites and sulphurous anhydride.

The formation of pigmenting organic metal sulphides is due to the fact that chlorhexidine is a protein-denaturing agent. In fact, the denaturation of proteins that are in the teeth-covering film, through the cleavage of sulphide bridges, causes the formation of sulphhydryl groups that may react with iron to form brown-pigmenting products.

According to the above-mentioned knowledge and further experimental studies, the applicant has considered different substances that could interfere with the formation of a brownish pigmentation on teeth. Among the different attempts, there was that one to associate chlorhexidine with iron complexing agents, but the extreme reactivity of chlorhexidine has prevented from going on with such researches in that direction. From the literature it is shown that iron responsible for such pigmentations is trivalent iron and not the bivalent one, and so further researches have been turned towards raw materials having reducing activities and thus being able to reduce the ferric iron into ferrous iron. It has also been shown that even the combination of chlorhexidine with ascorbic acid involves a fair reduction of trivalent iron to bivalent iron, but in order to carry this reduction further, it has been thought of adding to said combination a raw material selected from the group of sulphites, bisulphites and sulphurous anhydride. As a matter of fact, these substances inhibit Maillard reactions and therefore are suitable for solving the technical problem on which the present invention is based.

From further researches and experimentations carried out, it has been surprisingly found that the best results are obtained by adding sodium metabisulphite to the collutory based on chlorhexidine and ascorbic acid.

The presence of sodium metabisulphite in the collutory according to the present invention, apart from contrasting the Maillard reactions, has the further advantage of allowing to obtain a stable aqueous solution of ascorbic acid by preventing it from oxidation.

The collutory according to the present invention has the further advantage of providing also for the addition of sodium citrate to keep the pH thereof in a range between 5,7 and 6,3 at which chlorhexidine has the greatest activity.

Further advantages and features of the collutory according to the present invention will be clear to those skilled in the art from the following operative examples.

### EXAMPLE 1

In a volumetric 1 liter-flask some mls of a 20 wt/vol.% chlorhexidine digluconate solution furnished by Eingenmann & Veronelli SpA were introduced with a bulb pipette. Separately some grams of ascorbic acid and some grams of sodium metabisulphite as furnished by BASF were exactly weighed with an analytical balance. These substances were introduced in a beaker to which about 100 ml of purified water were added with a consequent rapid solubilization of the contents. The so obtained solution was added to the previously prepared first solution contained in the volumetric flask. A third solution was prepared by introducing in a 100 ml beaker 30 g of Cremophor (hydrogenated castor oil) furnished by Ingeman Veronelli as well as desired amounts of flavoring substances. This solution was added to the one contained in the volumetric flask wherein also 100 g of xylitol were introduced. After having brought it to a desired volume with purified water, the content of the volumetric flask was subjected to magnetic stirring and its pH was brought to 6 by adding sodium hydroxide furnished by Sigma.

With the final solution contained in the volumetric flask, by suitably varying the relative amounts of solutes, collutories A, B and C were produced having the following compositions:
A: chlorhexidine 0.12%, ascorbic acid 0.6% and sodium metabisulphite 0.3%;
B: chlorhexidine 0.2%, ascorbic acid 0.5% and sodium metabisulphite 0.3%;
C: chlorhexidine 0.3%, ascorbic acid 1% and sodium metabisulphite 0.5%;

The so prepared three collutories were tested on free subjects according to a standard protocol to be followed, that related to the diet, the mouthwash duration, the daily number of mouthwashes and the duration of the test. Collutories B and C were tested for 10 days.

Collutory A, containing a lower percentage of chlorhexidine, was tested for one month and produced a pigmentation on 2% of subjects only.

Collutory B produced a pigmentation on 1.5% of subjects only and collutory C on 2.5% of subjects only.

### EXAMPLE 2

With the solution prepared according to the Example 1 the following collutories were prepared by suitably varying the relative amounts:
D: chlorhexidine 0.05%, ascorbic acid 0.5%, sodium metabisulphite 0.2%
E: chlorhexidine 0.12%, ascorbic acid 0.3%, sodium metabisulphite 0.3%
F: chlorhexidine 0.2%, ascorbic acid 0.5%, sodium metabisulphite 0.5%.

The so prepared three collutories were tested according to the procedures specified in Example 1.

Collutory D produced pigmentation on 2% of subjects only. Collutory E produced pigmentation on 1.5% of subjects only. Collutory F on 1.7% of subjects only.

The chlorhexidine-based collutory according to the present invention has the further advantage of being able to be added with a fluoride with a view to confer it the beneficial properties of fluorine. These properties are well known and thus do not require any further detailed description. The procedure for preparing the fluorine added collutory according to the present invention is described in detail in the following example.

### EXAMPLE 3

In a volumetric 1 liter-flask some mls of a 20% chlorhexidine digluconate solution supplied by Eingenmann & Veronelli SpA were introduced with a bulb pipette. Separately some grams of ascorbic acid and some grams of sodium metabisulphite supplied by BASF AG as well as some grams of sodium fluoride were exactly weighed with an analytical balance. These substances were introduced in a beaker to which about 100 ml of purified water were added with a consequent rapid solubilization of the contents. The so obtained solution was added to the previously prepared one contained in the volumetric flask. A third solution was prepared by introducing in a 100 ml beaker 30 g of Cremophor (hydrogenated castor oil) supplied by Ingeman Veronelli and desired amounts of flavoring substances. This solution was added to the one present in the volumetric flask wherein also 100 g of xylitol were introduced. After having brought the contents of the volumetric flask to a desired volume with purified water, the resulting solution was subjected to magnetic stirring and its pH was brought to 6 by adding sodium hydroxide supplied by Sigma. SpA.

With the final solution contained in the volumetric flask, by suitably varying the relative amounts of solutes, collutories G, H and I having the following compositions were produced:
G: chlorhexidine 0.12%, ascorbic acid 0.6%, sodium metabisulphite 0.3% and sodium fluoride 0.08%;
H: chlorhexidine 0.2%, ascorbic acid 0.5%, sodium metabisulphite 0.3% and sodium fluoride 0.08%;
I: chlorhexidine 0.3%, ascorbic acid 1%, sodium metabisulphite 0.5% and sodium fluoride 0.08%.

The so prepared three collutories were subjected to the usage tests specified in Example 1 wherefrom the following results were obtained. Collutory G produced pigmentation on 2.2% of subjects only. Collutory H produced pigmentation on 1.8% of subjects only. Collutory I produced pigmentation on 2% of subjects only.

## Claims

1. A collutory comprising from 0.05 to 0.30wt/vol.% of chlorhexidine, from 0.1 to 1wt/vol% of ascorbic acid and from 0.1 to 0.5wt/vol.% of sodium metabisulphite.

2. A collutory according to claim 1, further comprising an alkali metal fluoride.

3. A collutory according to claim 1, further comprising from 0,05 to 0,1wt/vol.% of sodium fluoride.

## Patentansprüche

1. Mund- und Gurgellösung, die zwischen 0,05 und 0,30 % Gew./Vol. Chlorhexidin, zwischen 0,1 und 1 % Gew./Vol. Ascorbinsäure und zwischen 0,1 und 0,5 % Gew./Vol. Natriummetabisulfit enthält.

2. Mund- und Gurgellösung nach Anspruch 1, die weiterhin ein Alkalimetallfluorid enthält.

3. Mund- und Gurgellösung nach Anspruch 1, die weiterhin zwischen 0,05 und 0,1 % Gew./Vol. Natriumfluorid enthält.

## Revendications

1. Un collutoire comprenant de 0,05 à 0,03 % en poids/volume de chlorhexidine, de 0,1 à 1 % en poids/volume de acide ascorbique et de 0,1 à 0,5 % en poids/volume de métabisulfite de sodium.

2. Un collutoire selon la revendication 1, comprenant en outre un fluorure du métal alcalin.

3. Un collutoire selon la revendication 1, comprenant en outre de 0,05 à 0,1 % en poids/volume de fluorure de sodium.
